# EUROPEAN PATENT APPLICATION

(11) **EP 0 568 155 A1**
(43) Date of publication of application: **03.11.1993**
(21) Application number: 93201201.6
(22) Date of filing: 27.04.1993
(51) Int. Cl.: A61K 49/04

(54) **X-ray contrast formulations containing film-forming materials**

(30) Priority: 01.05.1992 US 877690
(71) Applicant: NYCOMED IMAGING AS, N-0485 Oslo (NO)
(72) Inventor: Illig, Carl R., c/o Sterling Winthrop Inc., New York, New York 10016 (US); Toner, John L., c/o Sterling Winthrop Inc., New York, New York 10016 (US)
(74) Representative: Woodman, Derek

(57) **Abstract**

An X-ray contrast formulation for oral or retrograde examination comprising:
a polymeric material capable of forming a coating on the gastrointestinal tract, said polymeric material having atoms containing polarizable electrons thereon, in combination with a divalent cation; and
a nonionic X-ray contrast producing agent having the formula: 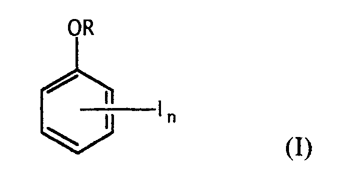
wherein R is a substituted or unsubstituted alkyl group containing from 2 to 8 carbon atoms wherein said substituents are selected from the group consisting of C₁-C₆ alkyl, hydroxy and alkoxy; and n is 1 to 5;
in a pharmaceutically acceptable carrier and a method for its use in diagnostic radiology of the gastrointestinal tract. The preferred contrast agent is the sec-octyl ether of 2,4,6-triodophenol.

## Description

This invention relates to an X-ray contrast formulation for oral or retrograde administration to a mammal comprising a nonionic X-ray contrast producing agent and a polymeric film-forming material.

Roentgenographic examination utilizing X-rays and computed tomography (hereinafter CT) scans of fractures and other conditions associated with the skeletal system is routinely practised without the use of contrast agents. X-ray visualization of organs containing soft tissue, such as the gastrointestinal (hereinafter GI) tract, requires the use of contrast agents which attenuate X-ray radiation. D. P. Swanson et al in "Pharmaceuticals In Medical Imaging", 1990, MacMillan Publishing Company, provides an excellent background in medical imaging utilizing contrast agents and compositions therewith.

The most widely used contrast agents for the visualization of the GI tract is barium sulfate administered as a suspension orally or rectally as an enema. (See, for example, U.S. Patent Nos.: 2,659,690; 2,680,089; 3,216,900; 3,235,462; 4,038,379 and 4,120,946) Notwithstanding its relatively good contrast characteristics, negligible absorption from the GI tract following oral or rectal administration and speedy excretion from the body, barium sulfate has certain disadvantages. In the presence of intestinal fluids it lacks homogeneity and poorly adheres to mucus membranes which can result in poor X-ray images. In the colon, when administered as an enema, it flocculates and forms irregular clumps with fecal matter.

Iodinated organic compounds have also been used as contrast agents since the iodine atom is an effective X-ray absorber. They have the most versatility and are utilized in the widest variety of procedures. They are very absorptive of X-rays with which the iodine interacts and produce a so-called photoelectric effect which is a large magnification in contrast caused by the photons stopped in the iodine-containing medium. The magnification of contrast exceeds the level that would be expected from relative changes in density. Because of this magnification, relatively low concentrations of the contrast agent can be utilized. (For iodinated agents see, for example, U.S. Patent Nos.: 2,786,055; 3,795,698; 3,360,436; 3,574,718, 3,733,397; 4,735,795 and 5,047,228.)

The desiderata for an ideal GI contrast agent includes: good toxicological profile; the ability to fill the entire bowel/lumen and evenly coat the gut mucosa so that the presence of the bowel is detectable when the lumen is not distended; palatability and nonirritation to the intestinal mucosa; and passing through the GI tract without producing artifacts or stimulating vigorous intestinal peristalsis.

These requirements were addressed by many investigators and their efforts resulted in great improvements over the years. The requirement of evenly coating the gut mucosa with and sufficiently adhering thereto, a contrast agent to effectively cover the walls of the intestines proved to be rather difficult. Without meeting these requirements it is impossible to obtain X-ray pictures of high precision. To that end, the use of certain polymer additives were proposed as illustrated hereunder.

U.S. Patent No. 4,069,306 discloses an X-ray contrast preparation which is said to adhere to the walls of body cavities. The preparation comprises a finely divided water-insoluble inorganic X-ray contrast agent and minute particles of a hydrophilic polymer which is insoluble in water but is water-swellable. The body cavity is supplied with such preparation suspended in water. The X-ray contrast agent is present in admixture with and/or enclosed in and/or adhered to said minute polymer particles.

U.S. Patent No. 4,120,946 discloses a pharmaceutical composition for barium opacification of the digestive tract, comprising colloidal barium sulfate and a polyacrylamide in an aqueous vehicle. The polyacrylamide forms a viscous solution at low concentration which makes it possible to maintain the barium sulfate in suspension and at the same time permit good adherence of the preparation to the walls of the organ which it is desired to X-ray.

U.S. Patent No. 5,019,370 discloses a biodegradable radiographic contrast medium comprising biodegradable polymeric spheres which carry a radiographically opaque element, such as iodine, bromine, samarium and erbium The contrast medium is provided either in a dry or liquid state and may be administered intravenously, orally and intra-arterially.

While these polymeric materials greatly enhance attachment of the contrast agent used therewith to the walls of organs for better visualization thereof, they do not provide a uniform coating thereon. As such, there is still a need for an improved X-ray imaging medium that uniformly coats the soft tissues subjected to diagnostic X-ray examination.

It is the object of the present invention to provide formulations for coating the gastrointestinal tract of mammals to form an effective radiopaque coating thereon by which diagnostic examination of the GI tract may be accomplished. To that end, a thin coating is formed on the inner surface of the GI tract effected by ingesting, prior to visualization by an X-ray emitting device, a polymeric film former, which has incorporated therein an X-ray contrast agent, capable of coating the GI tract. The removal of the coating occurs as a result of the normal turnover of cells, that is, within about 24 to 48 hours. Such formulations must meet several requirements: both the X-ray contrast agent and the film former must be nontoxic; must not contain leachable or digestible components that would deleteriously affect the patient; and the formulation must be capable of forming a film in the pH range of from about 5 to about 8.

The object of the present invention is achieved by a formulation comprising: an X-ray contrast agent; a polymeric material which is at least partially water soluble and contains polarizable or ionizable groups; and a divalent metal ion selected from the group consisting of Mg⁺⁺, Ca⁺⁺, Zn⁺⁺ and Ba⁺⁺ and a mixture thereof, which potentiates the effect of the polymeric material as a film former on the mucosa of the GI tract.

The contrast agent, polymeric film former and the divalent metal ion are incorporated in a solid or liquid media for administration to a mammal for X-ray visualization of the GI tract.

According to the present invention therefore there is provided an X-ray contrast formulation for oral or retrograde examination comprising:
a polymeric material capable of forming a coating on the gastrointestinal tract, said polymeric material having atoms containing polarizable electrons thereon, in combination with a divalent cation; and
a nonionic X-ray contrast producing agent having the formula: 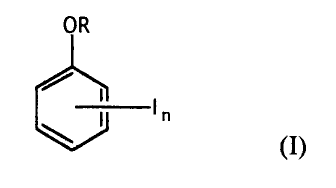
wherein R is a substituted or unsubstituted alkyl group containing from 2 to 8 carbon atoms wherein said substituents are selected from the group consisting of C₁-C₆ alkyl, hydroxy and alkoxy; and n is 1 to 5;
in a pharmaceutically acceptable carrier.

Preferred contrast agents of the present invention have the formula: 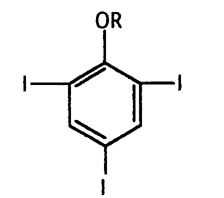
wherein R is an alkyl group containing from 4 to 8 carbon atoms, most preferably a secondary alkyl group.

The most preferred contrast agent utilized by the present invention is the sec-octyl ether of 2,4,6-triiodophenol having the formula: 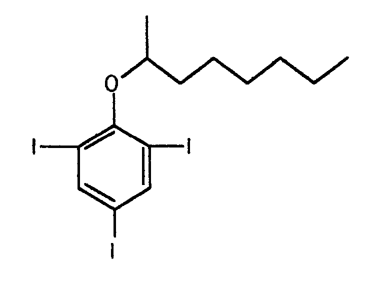
In another aspect of the invention there is provided a method of carrying out X-ray examination of the gastrointestinal tract of a patient, comprising the oral or rectal administration to the patient of an X-ray formulation as herein described in a pharmaceutically acceptable carrier.

The compounds are readily synthesizable by methods known in the prior art and as described hereinafter. The compounds' desirable properties include: sufficient iodine content for producing adequate imaging; ability to coat the mucosal lining of the GI tract giving good imaging quality; and superior toxicological profile.

The formulations may be in the form of solids, dispersions, colloids or suspensions, however, it is preferred to use emulsions as the preferred embodiment.

The contrast agents utilized in the formulations of the present invention are slightly soluble in water, having a partition coefficient equal to or greater than 10. This degree of solubility allows the formation of stable formulations in the form of dispersions, emulsions, suspensions when the formulations contain the requisite excipients. The term "stable" means that there is no separation of the ingredients contained in the formulations after oral or rectal administration thereof and during radiological examination of the GI tract. The slight solubility of the contrast agents in aqueous media permits diffusion of the contrast agents into the intestinal mucosa and secretions thereby forming a coating on the intestines. On the other hand, due to their slight solubility, the absorption of the contrast agent into the intestinal walls is minimal which reduces the possibility of toxic side effects.

The polymers that were found to be suitable for forming a thin coating on the GI tract can be classified as anionic, cationic and neutral polymers, having atoms containing polarizable electrons, in particular, selected from the group consisting of oxygen, nitrogen and sulfur. U. S. Patent No. 4,623,539, pertains to such polymers.

### I. Anionic Polymers

The anionic polymers carry negative charges in the ionized form and are capable of binding to cell surfaces mainly by electrostatic forces. Suitable anionic polymers include the following: 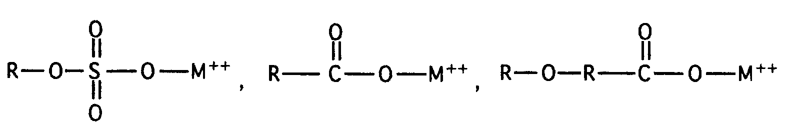
wherein
R is the polymeric chain; 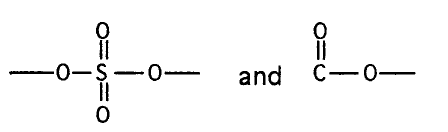
are anionic ligands; and
M⁺⁺ is a divalent cation.

Specific anionic polymers useful in the practice of the present invention include:
(1) Sulfated polysaccharides of the formula: 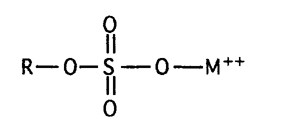 wherein R is 3,6-anhydro-D-galactose linked
   through C-4 to D-galactose; (kappa carrageenan)
   α-D-galactose units (1-3) linked; (lambda carrageenan)
   D-galactose
   3,6-anhydro-D-galactose; (iota carrageenan)
   D-galactose
   3,6-anhydro-L-galactose: (Agar - Agar)
   D-galactose
   3,6-anhydro-D-galactose; (Furcellaren)
   D-glucopyranose; (Laminarin sulfate)
   Galactan; and (Galactan sulfate)
   Galactosamino-glucuronans (Chondroitin sulfates);
   and
   M⁺⁺ is Mg⁺⁺, Ca⁺⁺, Zn⁺⁺, Ba⁺⁺ or mixtures thereof.
(2) Carboxylated polysaccharides of the formula: 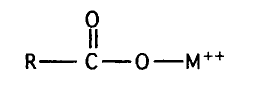 wherein R is D-galacturonoglycan; and (Pectin)
   anhydro-D-mannuronic acid
   and anhydro-L-guluronic acid (Algin)
   residues; and
   M⁺⁺ is Mg⁺⁺, Ca⁺⁺, Zn⁺⁺, Ba⁺⁺ or mixtures thereof.
(3) Cellulose derivatives of the formulae: 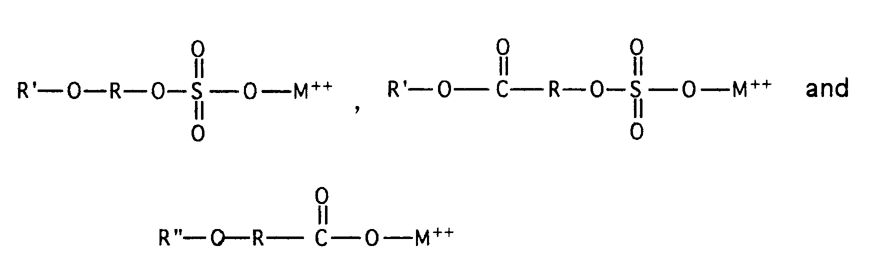 wherein R is an anhydroglucose residue;
   R' is CH₃, C₂H₅ or C₃H₇;
   R'' is CH₃ or C₂H₅; and
   M⁺⁺ is Mg⁺⁺, Ca⁺⁺, Zn⁺⁺, Ba⁺⁺ or mixtures thereof.
   Examples of cellulose derivatives include: sodium ethylcellulose sulfate, sodium cellulose acetate sulfate and sodium carboxymethyl cellulose.
(4) Sulfated, sulfonated or carboxylated synthetic polymers of the formula: 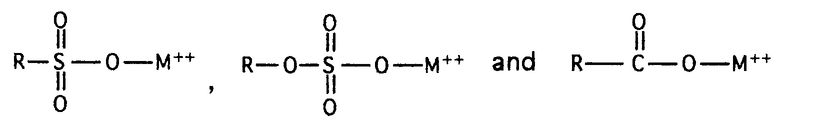 wherein
   R is an aliphatic or aromatic hydrocarbon, such as polystyrene, poly(sulfon) resin or carboxylated (poly) vinyl; and
   M⁺⁺ is Mg⁺⁺, Ca⁺⁺, Zn⁺⁺, Ba⁺⁺ or mixtures thereof.

### II Cationic Polymers

The cationic polymers carry positive charges in the ionized form. Suitable polymers for practising the present invention include: dermatan sulfate, keratosulfate, hyaluronic acid, heparin and chitin.

### III Neutral Polymers

Neutral polymers having polarizable electrons such as oxygen, nitrogen, sulfur, fluoride, chloride, bromide and iodide are also suitable for practising the present invention. In the presence of a cation, such as Mg⁺⁺, Ca⁺⁺, Zn⁺⁺ or Ba⁺⁺, the polymers are partially polarized thereby providing intermolecular interactions between the polymer and the intestinal wall. Examples of these polymers include:
(a) Polysaccharides, such as starch, glycogen, glucan, fructans, mannans, galactomannas, glucomannas, galactans, xylans, glycuranans, dextran and starch amylose;
(b) Cellulose derivatives, such as methylcellulose, hydroxyethylcellulose, ethylhydroxyethyl cellulose, hydroxypropyl methylcellulose and hydroxypropyl cellulose; and
(c) Synthetic polymers, such as polyvinylpyrrolidone, polyvinyl alcohol and ethylene oxide polymers.

The contrast agent is incorporated in the polymeric material along with the divalent cation by any suitable techniques, such as by mixing, blending, precipitating or by enclosing the contrast agent into minute polymeric particles.

The contrast agent, polymeric material and divalent cation blend is then formulated for administration using physiologically acceptable carriers or excipients in a manner within the skill of the art. The contrast agent with the addition of pharmaceutically acceptable aids (such as surfactants and emulsifiers) and excipients may be suspended or partially dissolved in an aqueous medium resulting in a dispersion, solution, suspension or emulsion. Alternatively, the contrast agent, polymeric material and divalent cation may be formulated into a solid form, such as tablets or capsules.

Formulations of the present invention comprise the following pharmaceutically acceptable components based on % w/v:

| | |
|---|---|
| Non-aqueous phase | 1 - 45 |
| Polymeric Material | 0.001 - 15 |
| Divalent Cation | 0.001 - 15 |
| Contrast Agent | 0.001 - 75 |
| Excipient | 0 - 20 |
| Aids (Surfactants/Emulsifiers) | 0.01 - 10 |
| Water | q.s. to 100 |

The nonaqueous phase comprises vegetable oils such as safflower oil; non-metabolizing fat substituents, such as Simpless; fluorinated hydrocarbons, such as perfluorodecaline; mineral oil and simethicone.

The contrast agent is selected from the group of compounds of formula 1, preferably compounds where R is an alkyl group containing from 4 to 8 carbon atoms.

Excipients advantageously used in the formulations include viscosity mediating and stabilizing agents, such as microcrystalline cellulose, ethylcellulose, hydroxypropyl methylcellulose and gum arabic. Physiologically acceptable substances may also be included, such as sodium citrate, sodium chloride, therapeutic substances, antacid substances and flavoring agents. The inclusion of antimicrobial/antiseptic agents such as methyl parahydroxybenzoate, ethyl parahydroxybenzoate, propyl parahydroxybenzoate, benzoic acid or sorbic acid may also be desirable in some formulations.

As known by those skilled in the art, surfactants or emulsifiers can reduce the interfacial tension between two immiscible phases, i.e., oil-in-aqueous medium. These agents can be used alone or in combination with other emulsifying agents and surfactants. For example, Dow Corning Medical Antifoam AF, which is a composition of 30% w/v polydimethylsiloxane and silica aerogel, 14% w/v stearate emulsifiers and 0.075% w/v sorbic acid, the balance being water, may be used by itself. Intralipid, which is an emulsion of fatty acids needs the presence of a suspending agent for it to form an acceptable emulsion with contrast agents of the present invention. The amount of such surfactants may be in the range of from 0.01 to about 10% w/v of the aqueous formulations, although the amount, in general, is kept as low as possible, preferably in the range of 0.05 to 2% w/v. The surface active agents may be cationic, anionic, nonionic, zwitterionic or a mixture of two or more of these agents.

Suitable cationic surfactants include cetyl trimethyl ammonium bromide and dodecyl dimethyl ammonium bromide. Suitable anionic agents include sodium lauryl sulphate, sodium heptadecyl sulphate, alkyl benzene-sulphonic acids and salts thereof, sodium butylnapthalene sulfonate, and sulphosuccinates. Zwitterionic surface-active agents are substances that when dissolved in water behave as diprotic acids and, as they ionize, behave both as a weak base and a weak acid. Since the two charges on the molecule balance each other out they act as neutral molecules. The pH at which the zwitterion concentration is maximum is known as the isoelectric point. Compounds, such as certain amino acids having an isoelectric point at the desired pH of the formulations of the present invention are useful in practising the present invention.

However in preparing the formulations of the present invention it is preferred to use nonionic emulsifiers or surface active agents which, similarly to the nonionic contrast agents, possess a superior toxicological profile to that of anionic, cationic or zwitterionic agents. In the nonionic emulsifying agents the proportions of hydrophilic and hydrophobic groups are about evenly balanced. They differ from anionic and cationic surfactants by the absence of charge on the molecule and, for that reason, are generally less irritant than the cationic or anionic surfactants. Nonionic surfactants include carboxylic esters, carboxylic amides, ethoxylated alkylphenols and ethoxylated aliphatic alcohols.

One particular type of carboxylic ester nonionic surface active agents are the partial, for example mono-, esters formed by the reaction of fatty and resin acids, for example of about 8 to about 18 carbon atoms, with polyhydric alcohols, for example glycerol, glycols such as mono-, di-, tetra- and hexaethylene glycol, sorbitan, and the like; and similar compounds formed by the direct addition of varying molar ratios of ethylene oxide to the hydroxy group of fatty acids.

Another type of carboxylic esters is the condensation products of a fatty and resin partial acid, for example mono-, esters ethylene oxide, such as fatty or resin acid esters of polyoxyethylene sorbitan and sorbitol, for example polyoxyethylene sorbitan, mono-tall oil esters. These may contain, for example, from about 3 to about 80 oxyethylene units per molecule and fatty or resin acid groups of from about 8 to about 18 carbon atoms. Examples of naturally occurring fatty acid mixtures which may be used are those from coconut oil and tallow while examples of single fatty acids are dodecanoic acid and oleic acid.

Carboxylic amide nonionic surface active agents are the ammonia, monoethylamine and diethylamides of fatty acids having an acyl chain of from about 8 to about 18 carbon atoms.

The ethoxylated alkylphenol nonionic surface active agents include various polyethylene oxide condensates of alkylphenols, especially the condensation products of mono-alkylphenols or dialkylphenols wherein the alkyl group contains about 6 to about 12 carbon atoms in either branched chain or particularly straight chain configuration, for example, octyl cresol, octyl phenol or nonyl phenol, with ethylene oxide, said ethylene oxide being present in amounts equal to from about 5 to about 25 moles of ethylene oxide per mole of alkylphenol.

Ethoxylated aliphatic alcohol nonionic surface active agents include the condensation products of aliphatic alcohols having from about 8 to 18 carbon atoms in either straight chain or branched chain configuration, for example oleyl or cetyl alcohol, with ethylene oxide, said ethylene oxide being present in equal amounts from about 30 to about 60 moles of ethylene oxide per mole of alcohol.

Preferred nonionic surface active agents include:
(a) Sorbitan esters (sold under the trade name Span) having the formula: 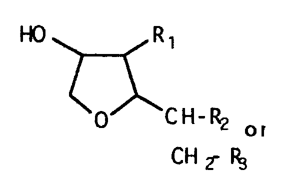 wherein
   R₁ = R₂ = OH, R₃ = R for sorbitan monoesters,
   R₁ = OH, R₂ = R₃ = R for sorbitan diesters,
   R₁ = R₂ = R₃ = R for sorbitan triesters,
   where R = (C₁₁H₂₃) COO for laurate,
   (C₁₇H₃₃) COO for oleate,
   (C₁₅H₃₁) COO for palmitate,
   (C₁₇H₃₅) COO for stearate;
(b) Polyoxyethylene alkyl ethers (i.e. Brijs) having the formula:
   CH₃(CH₂)ₓ(O-CH₂-CH₂)_{y}OH
   where (x + 1) is the number of carbon atoms in the alkyl chain, typically:
   12 lauryl (dodecyl)
   14 myristyl (tetradecyl)
   16 cetyl (hexadecyl)
   18 stearyl (octadecyl)
   and y is the number of ethylene oxide groups in the hydrophilic chain, typically 10-60;
(c) Polyoxyethylene sorbitan fatty acid esters, sold under the trade names of Polysorbates 20, 40, 60, 65, 80 & 85; and
(d) Polyoxyethylene stearates, such as:
   poly(oxy-1,2-ethanediyl)-α-hydro-ω-hydroxyoctadecanoate;
   polyethylene glycol monostearate; and
   poly(oxy-1,2-ethanediyl)-α-(1-oxooctadecyl)-ω-hydroxypolyethylene glycol monostearate.

Some of the contrast agents of the present invention can be prepared as described in U.S. Patent No. 2,622,100. It is preferred, however, to utilize the preparative methods described in the examples that follow.

The invention will therefore be more particularly described with reference to the following Examples but is in no way limited thereto.

### Example 1

### A. 2-Mesyloxyoctane

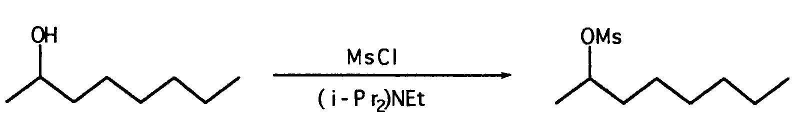

To 130 g (0.995 mol) 2-octanol and 207 ml (1.19 mol, 1.2 equiv) di-isopropylethylamine in 1.5 l dichloromethane at 0^{°}C under a CaSO₄ drying tube was added dropwise 84.4 ml (1.09 mol, 1.1 equiv.) methanesulfonyl chloride over 1 hour while keeping the internal temperature at less than 5^{°}C. The faintly yellow solution was stirred at 0^{°}C for an additional 1.5 h. The cold reaction solution was then washed with 2 x 1 l ice-cold 1 M HCl and 1 l ice-cold H₂O and brine and dried over MgSO₄. The residue was filtered through Celite and concentrated in vacuo below 35^{°}C to afford 208 g of a yellow oil. The yellow oil was placed under high-vacuum for 2 h at 25^{°}C to give 205 g of yellow oil. NMR spectra confirmed the desired title-product having only trace amounts of solvents present.

### B. 2,4,6-Triiodophenoxy-2-Octane

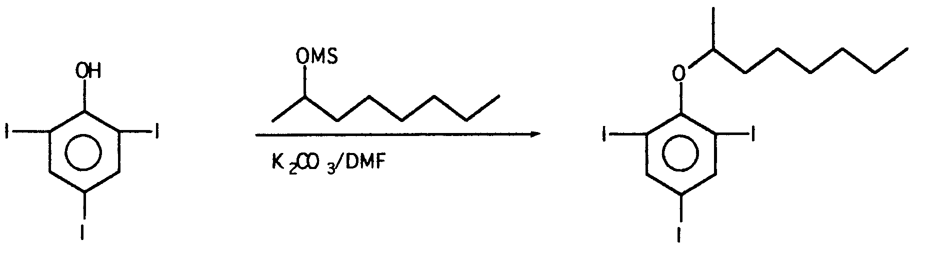

To a 5 l round bottom flask equipped with a mechanical stirrer was added, under nitrogen, 71.3 g (0.342 mol) 2-mesyloxyoctane (obtained in A), 193 g (0.410 mol, 1.2 equiv) 2,4,6-triiodophenol, 56.7 g (0.410 mol, 1.2 equiv.) anhydrous potassium carbonate and 2.00 l dimethylformamide (hereinafter DMF). The mixture was warmed slowly over one hour to reach a temperature of 55^{°}C to 60^{°}C and then stirred at 55^{°}C for 16 hours.

The temperature was raised to 65^{°}C and the mixture stirred for an additional 4 hours, then slowly cooled to room temperature and filtered through Celite to remove solids. The amber-colored DMF filtrate was extracted with 3 x 500 ml hexane to remove some of the product (first extract). TLC (25% EtOAc-hexane) indicated that the extract was the extremely pure title-product.

The DMF solution remaining after extraction with hexane was diluted with 9 l H₂O and 1 l 1 M NaOH. This mixture was extracted with 3 x 750 ml hexane (second extract). TLC showed that the extract was somewhat less pure than the first extract, but still only contained minor impurities.

The first extract and second extract were then separately washed with 2 x 500 ml 1 M NaOH, 500 ml H₂O, 500 ml saturated Na₂SO₃, 4 x 1 l H₂O, 1 l brine and dried over Na₂SO₄.

Concentration in vacuo followed by high vacuum afforded from the first extract 65.5 g of a faintly yellow oil (33% yield). NMR spectra confirmed the extremely pure title-product containing no measurable amount of other materials, such as unreacted mesylate.

The second extract afforded 92.2 g (46% yield) of a light amber-colored oil. NMR spectra confirmed a reasonably pure product having only trace amounts of impurities, such as mesylate.

The 65.5 g of product obtained from the first extract was filtered through 500 g of silica eluting with 6 l hexane to remove the trace of yellow color. Concentration in vacuo and under high vacuum and warming for 15 minutes afforded 64.7 g of the pure, colorless oil (99% recovery). NMR spectra confirmed the same very pure product as before.

The 92.2 g of product obtained from the second extract was subjected to the same procedure as the first extract. The product was found to be pure (98% recovery) without having a yellow color and without trace amounts of impurities, such as mesylate.

### Example 2

### 2,4,6-Triiodophenoxy-2-Butane

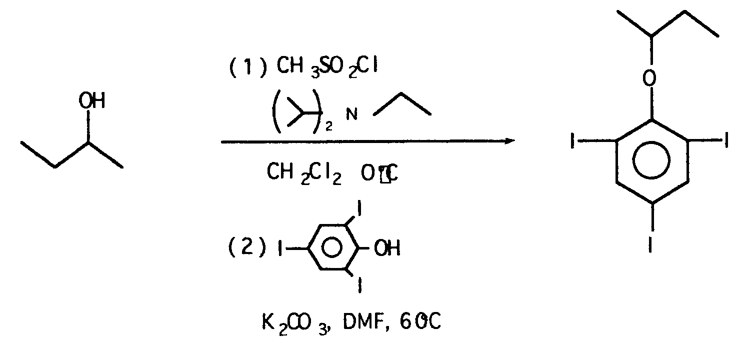

To a solution of 45.0 mmol 2-butanol (4.1 ml) in dichloromethane at 0^{°}C was added 1.2 equiv. (9.4 ml) diisopropylethylamine. After about 10 minutes, 1.1 equiv. (4.8 ml) of methanesulfonyl chloride was added slowly by syringe over about 10 min. The solution was stirred in an ice/water bath for 2.5 h, then poured over cold 5% HCl. The layers were separated and the organic layer was washed with cold 5% aqueous HCl and brine and dried over Na₂SO₄. The dried residue was dissolved in DMF (100 ml) and 50.0 mmol (23.6 g) 2,4,6-triiodophenol was added which was followed by the addition of 50.0 mmol (6.9 g) of potassium carbonate. (The solution at this point turned dark and was difficult to stir). Stirring continued for 17 h. The solution was then cooled, filtered through Celite using DMF. The so-obtained DMF-containing solution was twice extracted with hexane, diluted with 200 ml 0.1 M aqueous NaOH and again extracted twice with hexane. The hexane extracts were combined and washed with 2 x 50 ml 1 M NaOH, 2 x 50 ml H₂O and brine. The organic layer was dried over Na₂SO₄, filtered and the solvent removed in vacuo. Purification of the residue by flash column chromatography (silica, hexanes) gave 9.9 g of the title product as an oil.

An alternate method of making the compound of Example 2 is described in Example 3.

### Example 3

### 2,4,6-Triiodophenoxy-2-Butane

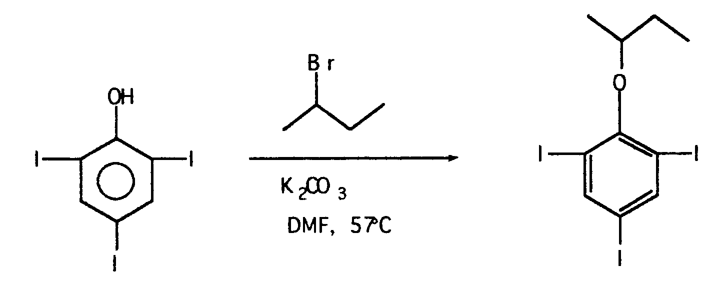

To a solution of 21.19 mmol (10.0 g) 2,4,6-triiodophenol in 40 ml (0.5 M) DMF at room temperature was added 2.0 equiv (4.6 ml) 2-bromobutane and 2.0 equiv. (5.86 g) potassium carbonate. The mixture was heated to 57^{°}C in an oil bath and stirred for 65 h. The mixture was then cooled, filtered through Celite by washing with DMF. The DMF-containing solution was extracted with hexane, diluted with 10% NaOH (100 ml) in H₂O, and extracted 3 times with hexane. The extracts were combined and washed twice with 1 M NaOH, twice with H₂O and twice with brine. The organic layer was dried over Na₂SO₄, filtered and the solvent was removed in vacuo. Purification by flash column chromatography (hexanes, silica) yielded 10.83 g of the title product in the form of an oil.

### Example 4

### 2,4,6-Triiodophenoxy-2-Hexane

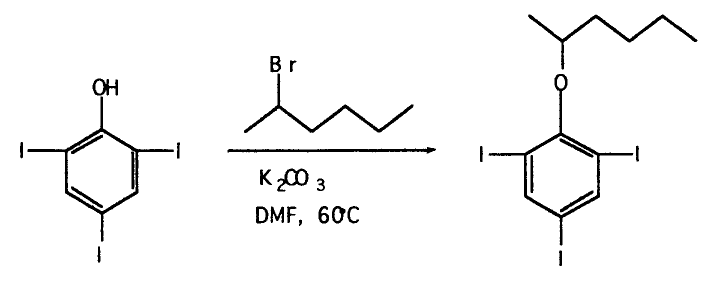

To a solution of 63.6 mmol (30g) 2,4,6-triiodophenol in 125 ml DMF (0.5 M) at room temperature was added 1.2 equiv. (10.8 ml) 2-bromohexane and 1.5 equiv. (13.2 g) potassium carbonate. The mixture was heated to 58°C over 1.5 h, then stirred 40 h. The reaction mixture was filtered through Celite using DMF. The volume of DMF was reduced to 200 ml by evaporation in vacuo. The mixture was extracted twice with hexane, diluted with 500 ml10% NaOH in H₂O and extracted again with hexane 3 times. The hexane extracts were then combined and washed twice with 1 M NaOH, twice with H₂O and once with brine. The organic layer was then dried over Na₂SO₄, filtered and the solvent was removed in vacuo. Flash column chromatography (hexanes, silica) yielded 31.5 g of the title compound in the form of an oil.

### Example 5

### 4-Iodophenoxy-2-Octane

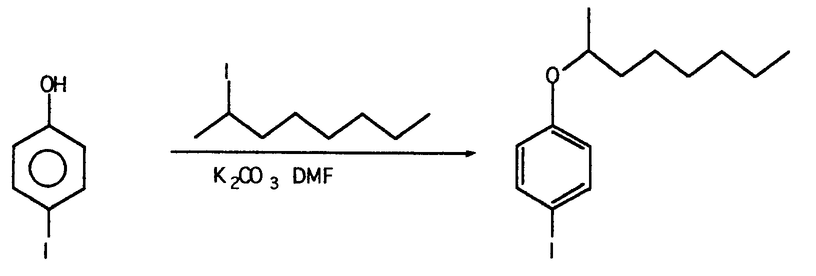

A mixture of 50.0 g (0.227 mol) 4-iodophenol, 45.4 g (0.189 mol) 2-iodooctane and 94.1 g (0.681 mol) potassium carbonate in 500 ml dry acetonitrile was heated to reflux under nitrogen and stirred for 20 h. The mixture was cooled and filtered through Celite and concentrated in vacuo. The brown residue was partitioned between 1 l hexanes and 500 ml 1 M NaOH. The hexane layer was then washed with 1 M NaOH (3 x 250 ml) saturated sodium sulfite (250 ml), water (250 ml) and brine (250 ml). The faintly yellow solution was dried over Na₂SO₄ and concentrated in vacuo to 34.3 g of a light yellow oil. The material in 60 ml hexane was passed through a 600 g pad of silica gel eluting with 3% ethyl acetate-hexanes until just prior to elution of the yellow color. Concentration and warming under high vacuum afforded 26.9 g (43%) of product as a mobile colorless oil.

Other compounds of formula 1 may be prepared using the reaction techniques described in Examples 1 through 5 using appropriate starting materials and reagents. It is to be understood that all reaction conditions, including choice of solvents, reaction atmosphere, reaction temperature, duration of the experiment and workup procedures, are chosen to be conditions standard for that reaction, which should be readily recognized by one skilled in the art. It is understood by one skilled in the art of organic synthesis that the functionality present on portions of the educt molecule must be compatible with the reagents and reactions.

Starting materials, reagents and solvents can be obtained from chemical suppliers, such as Aldrich, Baker and Eastman Chemical Companies, or they may be prepared by techniques known in the prior art.
Exemplary formulations of the present invention are as shown:

### Example 6

| | |
|---|---|
| Compound of Example 1 (114 mg l/ml) | 2.50 g (17.5% w/v) |
| Dow Corning Med. Antifoam AF emulsion | 3.50 g (35% w/v) |
| Galactan sulfate | 0.5 g (5% w/v) |
| Calcium lactate | 0.5 g (5% w/v) |
| Purified Water | q. s. to 10 ml |

### Example 7

| | |
|---|---|
| Compound of Example 1 (114 mg l/ml) | 2.50 g (17.5% w/v) |
| Safflower Oil | 2.00 g (20% w/v) |
| Tween-21 | 0.25 g (2.5% w/v) |
| Hydroxypropyl methylcellulose (4,000 cPs) | 2.50 g of 2% soln. |
| calcium lactate | 0.2 g (2% w/v) |
| Purified Water | q. s. to 10 ml |

### Example 8

| | |
|---|---|
| Compound of Example 1 (114 mg l/ml) | 2.50 g (17.5% w/v) |
| Mineral Oil | 0.50 g (5% w/v) |
| Heparin | 0.25 g (2.5% w/v) |
| Tween-21 | 0.25 g (2.5% w/v) |
| Calcium lactate | 0.25 g (2.5% w/v) |
| Purified Water | q. s. to 10 ml |

### Example 9

| | |
|---|---|
| Compound of Example 1 (114 mg l/ml) | 2.50 g (17.5% w/v) |
| Simplesse 100 (Nutrasweet Co.) | 3.00 g (30% w/v) |
| Calcium lactate | ∼0.5 (5% w/v) |
| Hydroxypropyl methylcellulose (4000 cPs) | 2.50 g of 2% soln.. |
| Purified Water | q. s. to 10 ml |

The dosages of the contrast agent used according to the method of the present invention will vary according to the precise nature of the contrast agent used. Preferably, however, the dosage should be kept as low as is consistent with achieving contrast enhanced imaging. By employing as small amount of contrast agent as possible, toxicity potential is minimized. For most contrast agents of the present invention dosages will be in the range of from about 0.1 to about 16.0 g iodine/kg body weight, preferably in the range of from about 0.5 to about 6.0 g iodine/kg of body weight, and most preferably, in the range of from about 1.2 to about 2.0 g iodine/kg body weight for regular X-ray visualization of the GI tract. For CT scanning the contrast agents of the present invention will be in the range of from about 1 to about 600 mg iodine/kg body weight, preferably in the range of from about 20 to about 200 mg iodine/kg body weight, and most preferably in the range of from about 40 to about 80 mg iodine/kg body weight.

The concentration of the contrast agent should be in the range of from about 0.001% w/v to about 75% w/v of the formulation, preferably from about 0.05% w/v to about 50% v/v and most preferably of from about 0.1 % w/v to about 20% w/v.

The concentration of the film forming polymeric material depends on the particular polymer used, however, it should be in the range of 0.01 to about 5% w/v or higher in combination with a divalent substance, such as calcium lactate, having a concentration range of 0.01 to 5% w/v. Dosage level of the polymeric material may be in the range of from about 2 to about 15 g/kg body weight or higher.

The formulations of the present invention possess very good adherence to the walls of the gastrointestinal tract by forming an essentially uniform coating thereon.

## Claims

1. An X-ray contrast formulation for oral or retrograde examination comprising:
a polymeric material capable of forming a coating on the gastrointestinal tract, said polymeric material having atoms containing polarizable electrons thereon, in combination with a divalent cation; and
a nonionic X-ray contrast producing agent having the formula: 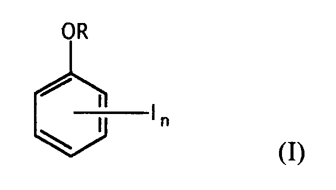 wherein R is a substituted or unsubstituted alkyl group containing from 2 to 8 carbon atoms wherein said substituents are selected from the group consisting of C₁-C₆ alkyl, hydroxy and alkoxy; and n is 1 to 5;
in a pharmaceutically acceptable carrier.

2. A formulation as claimed in claim 1 wherein the atoms having polarizable electrons are selected from the group consisting of oxygen, nitrogen and sulfur.

3. A formulation as claimed in claim 1 wherein the divalent cation is selected from the group consisting of Ca⁺⁺, Mg⁺⁺, Zn⁺⁺, Ba⁺⁺ and a mixture thereof.

4. A formulation as claimed in any one of the preceding claims wherein the X-ray producing agent is of the formula: 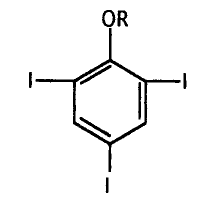 wherein R is an alkyl group containing from 4 to 8 carbon atoms.

5. A formulation as claimed in claim 4 wherein the X-ray producing agent is

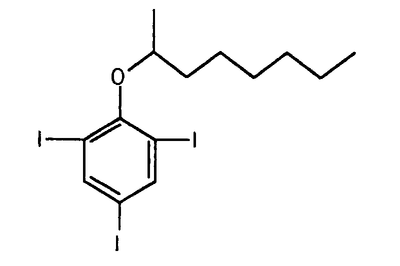

6. A formulation as claimed in claim 1 wherein the polymeric material is anionic having the formula: 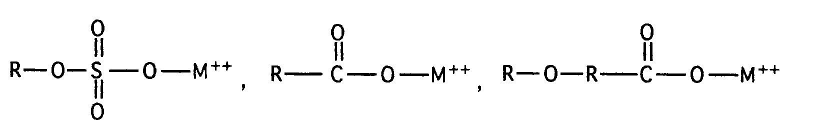 wherein
R is the polymeric chain; 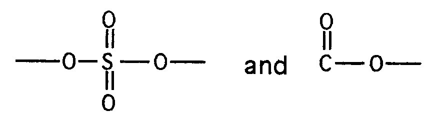 are anionic ligands; and
M⁺⁺ is a divalent cation.

7. A formulation as claimed in claim 6 wherein the anionic polymeric material is a sulfated polysaccharide having the formula: 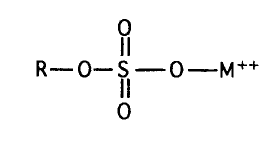 wherein R is 3,6-anhydro-D-galactose linked
through C-4 to D-galactose; (kappa carrageenan)
α-D-galactose units (1-3) linked; (lambda carrageenan)
D-galactose
3,6-anhydro-D-galactose; (iota carrageenan)
D-galactose
3,6-anhydro-L-galactose: (Agar - Agar)
D-galactose
3,6-anhydro-D-galactose; (Furcellaren)
D-glucopyranose; (Laminarin sulfate)
Galactan; and (Galactan sulfate)
Galactosamino-glucuronans (Chondroitin sulfates);
and
M⁺⁺ is Mg⁺⁺, Ca⁺⁺, Zn⁺⁺, Ba⁺⁺ or a mixture thereof.

8. A formulation as claimed in claim 6 wherein the anionic polymeric material is a carboxylated polysaccharide having the formula: 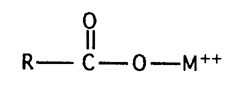 wherein R is D-galacturonoglycan; and (Pectin)
anhydro-D-mannuronic acid
and anhydro-L-guluronic acid (Algin)
residues; and
M⁺⁺ is Mg⁺⁺, Ca⁺⁺, Zn⁺⁺, Ba⁺⁺ or a mixture thereof.

9. A formulation as claimed in claim 6 wherein the anionic polymeric material is a cellulose derivative of the formula: 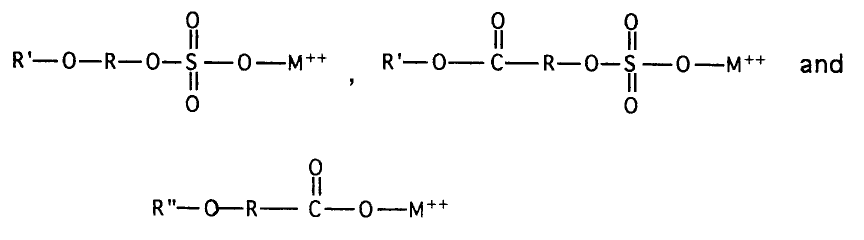 wherein R is an anhydroglucose residue;
R' is CH₃, C₂H₅ or C₃H₇;
R'' is CH₃ or C₂H₅; and
M⁺⁺ is Mg⁺⁺, Ca⁺⁺, Zn⁺⁺, Ba⁺⁺ or a mixture thereof.

10. A formulation as claimed in claim 1 wherein the polymeric material is cationic selected from the group consisting of: dermatan sulfate, keratosulfate, hyaluronic acid, heparin and chitin.

11. A formulation as claimed in claim 1 wherein the polymeric material is a polysaccharide.

12. A formulation as claimed in claim 1 wherein the polymeric material is a cellulose derivative.

13. A formulation as claimed in claim 1 wherein the polymeric material is polyvinylpyrrolidone, polyvinyl alcohol or an ethylene oxide polymer.

14. A formulation as claimed in claim 1 wherein the pharmaceutical carrier contains at least one surfactant.

15. A formulation as claimed in claim 14 wherein the surfactant is cationic.

16. A formulation as claimed in claim 14 wherein the surfactant is anionic.

17. A formulation as claimed in claim 14 wherein the surfactant is zwitterionic.

18. A formulation as claimed in claim 14 wherein the surfactant is nonionic.

19. A formulation as claimed in claim 18 wherein the nonionic surfactant is selected from the group consisting of carboxylic esters, carboxylic amides, ethoxylated alkylphenols and ethoxylated aliphatic alcohols, sorbitan esters, polyoxyethylene alkyl ethers and polyoxyethylene sorbitan fatty acid esters and polyoxyethylene stearates.

20. An orally/rectally administrable X-ray contrast formulation for X-ray examination of the gastrointestinal tract comprising an effective contrast-producing amount of an X-ray contrast agent having the formula 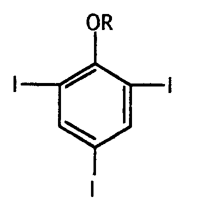 wherein
R is an alkyl group containing from 4 to 8 carbon atoms;
a polymeric material capable of forming a coating on the gastrointestinal tract, said polymeric material having atoms containing polarizable electrons thereon in combination with a divalent cation;
said X-ray contrast agent forming an emulsion in an aqueous medium comprising a surfactant.

21. A formulation as claimed in claim 20 wherein the X-ray contrast agent is

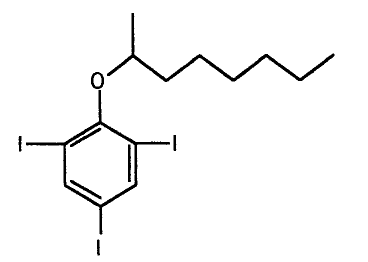

22. A formulation as claimed in any one of claims 14 to 21 wherein the surfactant comprises
30% w/v poly dimethylsiloxane and silica aerogel,
14% w/v stearate emulsifiers;
0.075% w/v sorbic acid; and
55.925% w/v water.

23. A formulation as claimed in any one of claims 14 to 22 wherein the surfactant is present from about 0.01% w/v to about 10% w/v in the aqueous formulation.

24. A method of carrying out X-ray examination of the gastrointestinal tract of a patient, comprising the oral or rectal administration to the patient of an X-ray contrast formulation comprising:
a polymeric material capable of forming a coating on the gastrointestinal tract, said polymeric material having atoms containing polarizable electrons thereon, in combination with a divalent cation; and
a nonionic X-ray contrast producing agent having the formula: 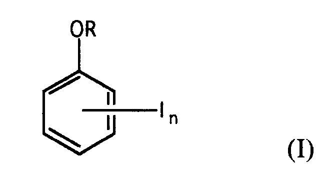 wherein R is a substituted or unsubstituted alkyl group containing from 2 to 8 carbon atoms wherein said substituents are selected from the group consisting of C₁-C₆ alkyl, hydroxy and alkoxy; and n is 1 to 5;
in a pharmaceutically acceptable carrier.

25. A method as claimed in claim 24 wherein the amount of contrast agent administered to said patient is in the range of from about 0.1 to about 16.0 g iodine/kg body weight for regular X-ray visualization of the gastrointestinal tract.

26. A method as claimed in claim 24 wherein the amount of contrast agent administered to said patient will be in the range of from about 1 to 600 mg iodine/kg body weight for CT scan visualization of the gastrointestinal tract.

27. A method as claimed in any one of claims 24 to 26 wherein the X-ray contrast agent is in the form of an emulsion.

28. A method as claimed in any one of claims 24 to 27 wherein the pharmaceutically acceptable carrier is as claimed in any one of claims 14 to 19.
composition further comprises a flavoring agent.

29. A method of carrying out X-ray examination of the gastrointestinal tract of a patient, said method comprising the oral or rectal administration to the patient of an X-ray contrast formulation as claimed in either of claims 20 and 21.

30. A method as claimed in claim 29 wherein the formulation is as claimed in either of claims 22 and 23.
